# EUROPÄISCHE PATENTSCHRIFT

(11) **EP 2 819 951 B1**
(45) Veröffentlichungstag und Bekanntmachung des Hinweises auf die Patenterteilung: **03.08.2016**
(21) Anmeldenummer: 13706047.1
(22) Anmeldetag: 27.02.2013
(51) Int. Cl.: C01D 15/00, C07C 309/10, H01G 11/62, H01M 10/052, H01M 10/0568, H01G 11/06, H01G 11/60

(54) **LEITSALZ FÜR LITHIUM-BASIERTE ENERGIESPEICHER**
CONDUCTING SALT FOR LITHIUM-BASED ENERGY STORAGE DEVICE
SEL CONDUCTEUR POUR DISPOSITIF DE STOCKAGE D'ÉNERGIE À BASE DE LITHIUM

(30) Priorität: 29.02.2012 DE 102012101670
(43) Veröffentlichungstag der Anmeldung: 07.01.2015
(73) Patentinhaber: Westfälische Wilhelms-Universität Münster, 48149 Münster (DE); Jacobs University Bremen gGmbH, 28759 Bremen (DE)
(72) Erfinder: RÖSCHENTHALER, Gerd-Volker, 28357 Bremen (DE); WINTER, Martin, 48149 Münster (DE); PASSERINI, Stefano, 89073 Ulm (DE); VLASOV, Katja, 68199 Mannheim (DE); KALINOVICH, Nataliya, 28755 Bremen (DE); SCHREINER, Christian, 86485 Biberbach (DE); SCHMITZ, Raphael Wilhelm, 48151 Münster (DE); MÜLLER, Romek Ansgar, 48455 Bad Bentheim (DE); SCHMITZ, René, 68163 Mannheim (DE); SCHEDLBAUER, Tanja, 94353 Haibach (DE); LEX-BALDUCCI, Alexandra, 48159 Münster (DE); KUNZE, Miriam, 37444 St. Andreasberg (DE)
(74) Vertreter: Michalski Hüttermann & Partner Patentanwälte mbB
(86) Internationale Anmeldenummer: PCT/EP2013/053949
(87) Internationale Veröffentlichungsnummer: WO 2013/127866

(56) Entgegenhaltungen:
- US-A1- 2006 276 670
- US-A1- 2007 066 822
- R. ARVAI ET AL: "New aryl-containing fluorinated sulfonic acids and their ammonium salts, useful as electrolytes for fuel cells or ionic liquids", JOURNAL OF FLUORINE CHEMISTRY, Bd. 129, Nr. 10, 1. Oktober 2008 (2008-10-01), Seiten 1029-1035, XP025468252, ISSN: 0022-1139, DOI: 10.1016/J.JFLUCHEM.2008.06.009 [gefunden am 2008-06-20]

## Beschreibung

Die Erfindung betrifft ein Leitsalz sowie einen Elektrolyten für Lithium-basierte Energiespeicher.

Die Lithium-Ionen-Technologie ist die führende Technologie auf dem Gebiet der wiederaufladbaren Batteriespeichersysteme für die portable Elektronik. Aufgrund ihrer hohen Zellspannung, ihrer überlegenen Energie- und Leistungsdichte sowie ihrer ausgesprochen niedrigen Selbstentladung haben Lithium-Ionen Batterien ein hohes Potential für diese Anwendungen. Derzeit wird in kommerziell erhältlichen Lithium-Ionen Batterien Lithiumhexafluorophosphat (LiPF₆) als Leitsalz verwendet. Lithiumhexafluorophosphat weist eine relativ hohe Leitfähigkeit auf, hat jedoch erhebliche Nachteile aufgrund einer geringen thermischen Stabilität und der Toxizität seiner Verbrennungsprodukte.

Daher gibt es intensive Bemühungen, alternative Lithiumsalze zu entwickeln, die LiPF₆ als Leitsalz ersetzen können. Die in den letzten Jahren entwickelten Lithiumsalze sind häufig komplexe Bor- oder Phosphorhaltige Anionen mit nicht-aromatischen Chelatbildnern wie Oxalat, beispielsweise das in der DE 198 29 030 C1 offenbarte Lithium-Bis(oxalato)borat (LiBOB). Nachteilig ist jedoch, dass Bis(oxalato)borat eine nur geringe Löslichkeit in den üblichen als Lösungsmittel in Elektrolyten verwendeten Carbonaten aufweist. Weiter weisen LiBOB-basierte Elektrolyte im Vergleich zu LiPF₆ eine geringere Leitfähigkeit, insbesondere bei niedrigen Temperaturen, und eine höhere Viskosität auf.

In Journal of Fluorine Chemistry, Bd. 129, Nr. 10, 2008, S. 1029-1035, werden neue Arylenthaltende fluorierte Sulfonsäuren und deren Ammoniumsalze offenbart, die als Elektrolyte für Brennstoffzellen oder ionische Flüssigkeiten verwendbar sind.

US 2006/276670 A1 offenbart Hydrofluoralkansulfonate der allgemeinen Formel R-O-CXH-CX₂-SO₃M, wobei R aus der Gruppe ausgewählt ist bestehend aus Alkylgruppen, funktionalisierten Alkylgruppen, und Alkenylgruppen; X ausgewählt ist aus der Gruppe bestehend aus Wasserstoff und Fluor, mit der Maßgabe, dass mindestens ein X Fluor ist; und M ein Kation ist.

US 2007/0066822 A1 offenbart Zusammensetzungen der Formel Z⁺A⁻, wobei Z⁺ ein Kation ist ausgewählt aus der Gruppe bestehend aus Pyridinium, Pyridazinium, Pyrimidinium, Pyrazinium, Imidazolium, Pyrazolium, Thiazolium, Oxazolium, Triazolium, und Phosphonium, sowie Ammoniumkationen mit spezifizierten Substituenten, und A- ausgewählt ist aus der Gruppe bestehend aus den drei Anionen R¹¹-CHF-CF₂-SO₃⁻, R¹²-CF₂-CF₂-SO₃⁻ und (R¹³-CHF-CF₂-SO₂)₂N⁻ mit spezifizierten Resten R¹¹, R¹² und R¹³, die als ionische Flüssigkeit verwendbar sind.

Trotz einer Vielzahl an Salzen und Lösungsmitteln wurde noch kein geeigneter Ersatz für LiPF₆ als Leitsalz in Carbonatgemischen gefunden. Daher besteht ein Bedarf an alternativen Lithiumsalzen für die Verwendung in Lithium-Ionen Batterien.

Der vorliegenden Erfindung lag daher die Aufgabe zu Grunde, eine Verbindung zur Verfügung zu stellen, die mindestens einen der vorgenannten Nachteile des Standes der Technik überwindet. Insbesondere lag der vorliegenden Erfindung die Aufgabe zu Grunde, eine als Leitsalz geeignete Lithiumverbindung zur Verfügung zu stellen.

Diese Aufgabe wird gelöst durch die Verbindung Lithium-1-trifluormethoxy-1,2,2,2-tetrafluor-ethansulfonat. Lithium-1-trifluormethoxy-1,2,2,2-tetrafluor-ethansulfonat wird auch als Lithium-1,2,2,2-tetrafluor-1-(trifluormethoxy)ethansulfonat bezeichnet.

Überraschend wurde gefunden, dass Lithium-1-trifluormethoxy-1,2,2,2-tetrafluor-ethansulfonat eine gute Lithium-Ionen Leitfähigkeit und eine hohe elektrochemische Stabilität aufweist. In vorteilhafter Weise ist Lithium-1-trifluormethoxy-1,2,2,2-tetrafluor-ethansulfonat in einem weiten Temperaturbereich als Leitsalz verwendbar. Weiter zeigen Zellen, in denen Elektrolyte, die Lithium-1-trifluormethoxy-1,2,2,2-tetrafluor-ethansulfonat als Leitsalz enthalten, verwendet wurden, eine gute Zyklenstabilität und Kapazitätsretention.

Darüber hinaus ist ein großer Vorteil von Lithium-1-trifluormethoxy-1,2,2,2-tetrafluor-ethansulfonat eine gute thermische Stabilität. Insbesondere ist von Vorteil, dass Lithium-1-trifluormethoxy-1,2,2,2-tetrafluor-ethansulfonat auch bei hohen Temperaturen keine oder fast keine Anzeichen einer Zersetzung zeigt. So wurde in thermischen Alterungsversuchen festgestellt, dass bei längerer Lagerung eines Elektrolyten umfassend Lithium-1-trifluormethoxy-1,2,2,2-tetrafluor-ethansulfonat in Karbonat-Lösungsmitteln bei 95 °C keine Karbonatzersetzungsprodukte im Elektrolyten feststellbar sind, welche beim thermischen Lagern von LiPF₆ in Karbonat-Mischungen auftreten.

Hierdurch kann im Vergleich zur Verwendung von LiPF₆ als Leitsalz eine erhebliche Verbesserung der Betriebssicherheit zur Verfügung gestellt werden. In insbesondere vorteilhafter Weise ermöglicht dies eine Verwendung im Hochtemperaturbereich. Lithium-1-trifluormethoxy-1,2,2,2-tetrafluor-ethansulfonat zeigt insbesondere eine geringere Toxizität der Verbrennungsprodukte als LiPF₆. Lithium-1-trifluormethoxy-1,2,2,2-tetrafluor-ethansulfonat ist daher in vorteilhafter Weise als Leitsalz für kommerzielle Lithium-Ionen Batterien geeignet. Weiterhin erwies sich in vorteilhafter Weise auch die anodische Stabilität eines Elektrolyten umfassend Lithium-1-trifluormethoxy-1,2,2,2-tetrafluor-ethansulfonat in Karbonatgemischen auf poliertem Platin mit über 5 V als ausreichend um mit den gängigen Kathodenmaterialien zyklisiert werden zu können.

Ein weiterer Gegenstand der vorliegenden Erfindung betrifft die Verwendung von Lithium-1-trifluormethoxy-1,2,2,2-tetrafluor-ethansulfonat in Lithium-basierten Energiespeichern, insbesondere als Leitsalz. Insbesondere ist Lithium-1-trifluormethoxy-1,2,2,2-tetrafluor-ethansulfonat als Leitsalz in Elektrolyten für Lithium-basierte Energiespeicher verwendbar. Primäre und sekundäre Lithium-basierte Energiespeicher sind vorzugsweise ausgewählt aus der Gruppe umfassend Lithium-Batterien, Lithium-Ionen-Batterien, Lithium-Ionen-Akkumulatoren, Lithium-Polymer-Batterien und/oder Lithium-Ionen-Kondensatoren. Insbesondere eignet sich Lithium-1-trifluormethoxy-1,2,2,2-tetrafluor-ethansulfonat als Leitsalz für eine Lithium-Ionen Batterie oder einen Lithium-Ionen Akkumulator.

In vorteilhafter Weise kann durch Verwendung von Lithium-1-trifluormethoxy-1,2,2,2-tetrafluor-ethansulfonat in Lithium-basierten Energiespeichern ein thermisch stabiles Leitsalz zur Verfügung gestellt werden. Hierdurch ist ein Lithium-basierter Energiespeicher in einem weiten Temperaturbereich verwendbar. Vorzugsweise ist Lithium-1-trifluormethoxy-1,2,2,2-tetrafluor-ethansulfonat in Elektrolyten für Lithium-basierte Energiespeicher in Konzentration im Bereich von ≥ 0,1 M bis ≤ 2 M, vorzugsweise im Bereich von ≥ 0,5 M bis ≤ 1,5 M, besonders bevorzugt im Bereich von ≥ 0,7 M bis ≤ 1,2 M, verwendbar. Insbesondere ist Lithium-1-trifluormethoxy-1,2,2,2-tetrafluor-ethansulfonat in einer Konzentration von 1 M verwendbar.

Der Ladungstransport in Lithium-basierten elektrochemischen Energiespeichern erfolgt über einen Elektrolyten. Das Leitsalz eines flüssigen Elektrolyten liegt daher vorzugsweise in einem Lösungsmittel gelöst vor. Der Elektrolyt umfasst Lithium-1-trifluormethoxy-1,2,2,2-tetrafluor-ethansulfonat vorzugsweise gelöst in einem organischen Lösungsmittel. Der Elektrolyt ist beispielsweise herstellbar durch Einbringen und Lösen von Lithium-1-trifluormethoxy-1,2,2,2-tetrafluor-ethansulfonat in ein Lösungsmittel oder ein Lösungsmittelgemisch.

Ein weiterer Gegenstand der Erfindung betrifft einen Elektrolyt für einen Lithium-basierten Energiespeicher umfassend Lithium-1-trifluormethoxy-1,2,2,2-tetrafluor-ethansulfonat. Elektrolyte für Lithium-basierte Energiespeicher umfassend Lithium-1-trifluormethoxy-1,2,2,2-tetrafluor-ethansulfonat als Leitsalz weisen insbesondere den Vorteil einer guten thermischen und elektrochemischen Stabilität auf.

Vorzugsweise umfasst der Elektrolyt ein organisches Lösungsmittel, eine ionische Flüssigkeit und/oder eine Polymermatrix. Vorzugsweise umfasst ein Elektrolyt ein organisches Lösungsmittel und Lithium-1-trifluormethoxy-1,2,2,2-tetrafluor-ethansulfonat. Es konnte festgestellt werden, dass Lithium-1-trifluormethoxy-1,2,2,2-tetrafluor-ethansulfonat in organischen Lösungsmitteln insbesondere Gemischen mit cyclischen oder linearen Carbonaten eine gute Löslichkeit aufweist. Dies ermöglicht in vorteilhafter Weise die Verwendung von Lithium-1-trifluormethoxy-1,2,2,2-tetrafluor-ethansulfonat in den in kommerziellen Lithium-Ionen Batterien eingesetzten Flüssigelektrolyten.

In bevorzugten Ausführungsformen ist das organische Lösungsmittel ausgewählt aus der Gruppe umfassend Ethylencarbonat, Propylencarbonat, Diethylcarbonat, Dimethylcarbonat, Ethylmethylcarbonat, Acetonitril, Glutaronitril, Adiponitril, Pimelonitril, gamma-Butyrolacton, gamma-Valerolacton, Dimethoxyethan, 1,3-Dioxolan, Methylacetat und/oder Mischung davon. Geeignete organische Lösungsmittel sind ausgewählt aus der Gruppe umfassend cyclische Carbonate wie Ethylencarbonat und Propylencarbonat und/oder lineare Carbonate wie Diethylcarbonat, Dimethylcarbonat und Ethylmethylcarbonat. Weitere geeignete organische Lösungsmittel sind ausgewählt aus der Gruppe umfassend gamma-Butyrolacton und/oder gamma-Valerolacton. Vorzugsweise ist das organische Lösungsmittel ausgewählt aus der Gruppe umfassend Ethylencarbonat, Diethylcarbonat, Dimethylcarbonat, Ethylmethylcarbonat, gamma-Butyrolacton, gamma-Valerolacton und/oder deren Mischungen.

Ein bevorzugtes Lösungsmittel ist Ethylencarbonat. Ethylencarbonat wird gemäß der IUPAC-Nomenklatur auch als 1,3-Dioxolan-2-on bezeichnet . Ethylencarbonat ist kommerziell erhältlich und weist einen hohen Siedepunkt und einen hohen Flammpunkt auf. Weiter vorteilhaft ist, dass Ethylencarbonat eine hohe Leitfähigkeit durch eine gute Salzdissoziation ermöglicht.

In einer bevorzugten Ausführungsform umfasst das organische Lösungsmittel eine Mischung aus Ethylencarbonat und wenigstens einem weiteren organischen Lösungsmittel, vorzugsweise ausgewählt aus der Gruppe umfassend gamma-Butyrolacton und/oder Diethylcarbonat. Bevorzugt umfasst das organische Lösungsmittel eine Mischung aus Ethylencarbonat und gamma-Butyrolacton. In Lösungsmittelgemischen umfassend Ethylencarbonat und gamma-Butyrolacton konnte in vorteilhafter Weise eine hohe Leitfähigkeit in einem Temperaturbereich von -20°C bis +60°C erreicht werden. Insbesondere können in Lösungsmittelgemischen umfassend gamma-Butyrolacton hohe Leitfähigkeiten in einem Temperaturbereich von -30°C bis +60°C erreicht werden. Geeignet sind beispielsweise binäre Mischungen von Carbonaten, insbesondere von Ethylencarbonat mit einem weiteren Carbonat, beispielsweise Diethylcarbonat, Dimethylcarbonat und/oder Ethylmethylcarbonat, insbesondere Diethylcarbonat.

Bevorzugt liegt das Verhältnis von Ethylencarbonat und dem wenigstens einen weiteren organischen Lösungsmittel, vorzugsweise gamma-Butyrolacton oder Diethylcarbonat, im Bereich von ≥ 1:99 bis ≤ 99:1, bevorzugt im Bereich von ≥ 1:9 bis ≤ 9:1, vorzugsweise im Bereich von ≥ 3:7 bis ≤ 1:1. Wenn nicht abweichend angegeben bezieht sich das angegebene Verhältnis auf die Gewichtsanteile der Lösungsmittel.

Bevorzugt sind auch ternäre Mischungen, umfassend wenigstens ein Carbonat als Lösungsmittel. Insbesondere bevorzugt sind Mischungen von Ethylencarbonat mit einem weiteren Lösungsmittel beispielsweise gamma-Butyrolacton oder Diethylcarbonat und einer Verbindung, die geeignet ist, eine sogenannte Solid Electrolyte Interphase (SEI), eine Feststoff-Elektrolyt-Grenzfläche, auszubilden. Der Elektrolyt kann daher ferner Additive, insbesondere filmbildende Elektrolytadditive, umfassen.

In bevorzugten Ausführungsformen umfasst der Elektrolyt eine Verbindung ausgewählt aus der Gruppe umfassend Chlorethylencarbonat, Fluorethylencarbonat, Vinylethylencarbonat, Ethylensulfit, Ethylensulfat, Propansulfonate, Sulfite, vorzugsweise Dimethylsulfit und Propylensulfit, Sulfate, optional mit F, Cl oder Br substituierte Butyrolactone, Phenylethylencarbonat, Vinylacetat und/oder Trifluorpropylencarbonat.

Von den Verbindungen auf Carbonat-Basis sind mit Chlor oder Fluor substituierte Carbonate bevorzugt, insbesondere Fluorethylencarbonat (FEC). Die Verbindungen können die Batterieleistung verbessern, beispielsweise die Kapazität oder die Zyklenlebensdauer. Insbesondere Fluorethylencarbonat kann zu einer verbesserten Langzeitstabilität einer Zelle führen.

Vorzugsweise enthält der Elektrolyt ein Additiv, insbesondere eine Verbindung ausgewählt aus der Gruppe umfassend Chlorethylencarbonat, Fluorethylencarbonat, Vinylethylencarbonat, Ethylensulfit, Ethylensulfat, Propansulfonate, Sulfite, vorzugsweise Dimethylsulfit und Propylensulfit, Sulfate, optional mit F, Cl oder Br substituierte Butyrolactone, Phenylethylencarbonat, Vinylacetat und/oder Trifluorpropylencarbonat, vorzugsweise Fluorethylencarbonat, im Bereich von ≥ 0,1 Gew.-% bis ≤ 10 Gew.-%, bevorzugt im Bereich von ≥ 1 Gew.-% bis ≤ 5 Gew.-%, vorzugsweise im Bereich von ≥ 2 Gew.-% bis ≤ 3 Gew.-%, bezogen auf das Gesamtgewicht des Elektrolyts.

Bevorzugt umfasst das organische Lösungsmittel eine Mischung aus Ethylencarbonat und wenigstens einem weiteren organischen Lösungsmittel, vorzugsweise ausgewählt aus der Gruppe umfassend gamma-Butyrolacton, gamma-Valerolacton oder Diethylcarbonat und Fluorethylencarbonat.

Bevorzugt liegt das Verhältnis von Ethylencarbonat und dem wenigstens einen weiteren organischen Lösungsmittel, vorzugsweise ausgewählt aus der Gruppe umfassend gamma-Butyrolacton, gamma-Valerolacton oder Diethylcarbonat, und einem Additiv, vorzugsweise Fluorethylencarbonat, im Bereich von ≥ 98:1:1 bis ≤ 1:1:98, bevorzugt im Bereich von ≥ 80:15:5 bis ≤ 15:80:5, vorzugsweise im Bereich von ≥ 4,5:4,5:1 bis ≤ 1:1:0,01. Wenn nicht abweichend angegeben bezieht sich das angegebene Verhältnis auf die Gewichtsanteile.

Besonders bevorzugt umfasst das organische Lösungsmittel eine Mischung aus Ethylencarbonat, gamma-Butyrolacton und Fluorethylencarbonat, insbesondere in einem Verhältnis von 4,5:4,5:1. In einem Lösungsmittelgemisch umfassend Ethylencarbonat, gamma-Butyrolacton und Fluorethylencarbonat in einem Verhältnis von 4,5:4,5:1 konnte in vorteilhafter Weise eine gute Leitfähigkeit in einem Temperaturbereich von -20°C bis +60°C erreicht werden.

Als vielversprechende Lösungsmittel haben sich weiterhin ionische Flüssigkeiten erwiesen, da diese eine hohe thermische wie elektrochemische Stabilität mit einer hohen ionischen Leitfähigkeit vereinen. Insbesondere ist dies vorteilhaft zur Verwendung mit Lithium-1-trifluormethoxy-1,2,2,2-tetrafluor-ethansulfonat. Bevorzugte ionische Flüssigkeiten umfassen ein Kation ausgewählt aus der Gruppe umfassend 1-Ethyl-3-methylimidazolium (EMI⁺), 1,2-Dimethyl-3-propylimidazolium (DMPI⁺), 1,2-Diethyl-3,5-dimethylimidazolium (DEDMI⁺), Trimethyl-*n*-hexylammonium (TMHA⁺), *N-*alkyl-*N-*methylpyrrolidinium (PYR_{1R}⁺), *N-*alkyl-*N-*methylpiperidinium (PIP_{1R}⁺) und/oder *N-*alkyl-*N-*methylmorpholinium (MORP_{1R}⁺) und ein Anion ausgewählt aus der Gruppe umfassend Bis(trifluormethansulfonyl)imid (TFSI⁻), Bis(pentafluorethansulfonyl)imid (BETI⁻), Bis(fluorsulfonyl)imid (FSI⁻), 2,2,2-Trifluor-*N-*(trifluormethansulfonyl)acetamid (TSAC⁻), Tetrafluorborat (BF₄⁻), Pentafluorethanetrifluoroborate (C₂F₅BF₃⁻), Hexafluorphosphat (PF₆⁻) und/oder Tri(pentafluorethan)trifluorphosphat ((C₂F₅)₃PF₃⁻). Bevorzugte *N-*alkyl-*N-*methylpyrrolidinium (PYR_{1R}⁺) Kationen sind ausgewählt aus der Gruppe umfassend N-butyl-N-methylpyrrolidinium (PYR₁₄⁺) und/oder N-methyl-N-propylpyrrolidinium (PYR₁₃⁺). Bevorzugte ionische Flüssigkeiten sind ausgewählt aus der Gruppe umfassend N-butyl-N-methylpyrrolidiniumbis(trifluoromethansulfonyl)imid (PYR₁₄TFSI) und/oder N-methyl-N-propylpyrrolidiniumbis(trifluoromethansulfonyl)imid (PYR₁₃TFSI).

Weitere geeignete Elektrolytmaterialien sind Polymerelektrolyte, wobei der Polymerelektrolyt als Gelpolymerelektrolyt oder fester Polymerelektrolyt vorliegen kann. Feste Polyelektrolyte zeigen gute Eigenschaften bezüglich der Anforderungen an zukünftige Akkumulatorgenerationen. Sie ermöglichen einen solvensfreien Aufbau, der einfach herzustellen und vielfältig in der Form ist. Darüber hinaus kann die Energiedichte gesteigert werden, denn es entfällt der Dreischichtaufbau aus Elektrolyt-Separator-Elektrolyt, sodass lediglich eine dünne Polymerfolie zwischen den Elektroden benötigt wird. Festelektrolyte sind in der Regel chemisch und elektrochemisch stabil gegenüber Elektrodenmaterialien und treten weiterhin nicht aus der Zelle aus. Gelpolymerelektrolyte umfassen meist ein aprotisches Lösungsmittel und eine Polymermatrix.

Bevorzugte Polymere für feste Polymerelektrolyte und Gelpolymerelektrolyte sind ausgewählt aus der Gruppe umfassend Homo- oder Copolymere von Polyethylenoxid (PEO), Polypropylenoxid (PPO), Polyvinylidenfluorid (PVdF), Polyvinylidenfluoridhexafluorpropylen (PVdF-HFP), Polyacrylnitril (PAN), Polymethylmethacrylat (PMMA), Polyethylmethacrylat (PEMA), Polyvinylacetat (PVAc), Polyvinylchlorid (PVC), Polyphophazene, Polysiloxane, Polyvinylalkohol (PVA) und/oder Homo- und (Block-) Copolymere umfassend funktionelle Seitenketten ausgewählt aus der Gruppe umfassend Ethylenoxid, Propylenoxid, Acrylnitril und/oder Siloxane.

In bevorzugten Ausführungsformen liegt die Konzentration an Lithium-1-trifluormethoxy-1,2,2,2-tetrafluor-ethansulfonat im Elektrolyten im Bereich von ≥ 0,1 M bis ≤ 2 M, vorzugsweise im Bereich von ≥ 0,5 M bis ≤ 1,5 M, besonders bevorzugt im Bereich von ≥ 0,7 M bis ≤ 1,2 M. In einer besonders bevorzugten Ausführungsform liegt die Konzentration an Lithium-1-trifluormethoxy-1,2,2,2-tetrafluor-ethansulfonat im Elektrolyten bei 1 M. In vorteilhafter Weise führen solche Konzentrationen an Lithium-1-trifluormethoxy-1,2,2,2-tetrafluor-ethansulfonat zu einer guten Leitfähigkeit.

Ein weiterer Gegenstand der Erfindung betrifft einen Lithium-basierten Energiespeicher, vorzugsweise eine Lithium-Batterie, Lithium-Ionen-Batterie, Lithium-Ionen-Akkumulator, Lithium-Polymer-Batterie oder Lithium-Ionen-Kondensator umfassend Lithium-2-trifluormethoxy-1,2,2,2-tetrafluor-ethansulfonat. Ein weiterer Gegenstand der Erfindung betrifft einen Lithium-basierten Energiespeicher, vorzugsweise eine Lithium-Batterie, Lithium-Ionen-Batterie, Lithium-Ionen-Akkumulator, Lithium-Polymer-Batterie oder Lithium-Ionen-Kondensator, umfassend einen erfindungsgemäßen Elektrolyten umfassend Lithium-1-trifluormethoxy-1,2,2,2-tetrafluor-ethansulfonat.

Die Lithium-basierten Energiespeicher eignen sich für alle Anwendungsgebiete, insbesondere auch für größere Systeme wie Automobile oder als stationäre Energiespeicher für erneuerbare Energien.

Lithium-1-trifluormethoxy-1,2,2,2-tetrafluor-ethansulfonat ist nach üblichen Synthesemethoden herstellbar. Bevorzugt ist ein Verfahren zur Herstellung von Lithium-1-trifluormethoxy-1,2,2,2-tetrafluor-ethansulfonat, umfassend die folgenden Schritte:
a) Umsetzung von 1,1,2-Trifluor-2-(trifluormethoxy)ethen mit Sulfuryldifluorid zu 1,2,2,2-Tetrafluor-1-(trifluormethoxy)-ethansulfonylfluorid, und
b) Umsetzung von 1,2,2,2-Tetrafluor-1-(trifluormethoxy)-ethansulfonylfluorid mit Lithiumhydroxid zu Lithium-1-trifluormethoxy-1,2,2,2-tetrafluor-ethansulfonat.

Bevorzugt erfolgt die Umsetzung von 1,1,2-Trifluor-2-(trifluormethoxy)ethen, CF₃OCF=CF₂, mit Sulfuryldifluorid, SO₂F₂, in Gegenwart von katalytischen Mengen an Tetramethylammoniumfluorid. Die Umsetzung erfolgt vorzugsweise unter Erwärmen, insbesondere bei einer Temperatur im Bereich von 50°C bis 60°C. Die anschließende Hydrolyse von 1,2,2,2-Tetrafluor-1-(trifluormethoxy)-ethansulfonylfluorid mit Lithiumhydroxid erfolgt vorzugsweise mit ethanolischer Lithiumhydroxidlösung.

Als Vorstufe erhält man in Schritt a) 1,2,2,2-Tetrafluor-1-(trifluormethoxy)-ethansulfonylfluorid. In vorteilhafter Weise ist die Umsetzung von 1,1,2-Trifluor-2-(trifluormethoxy)ethen mit Sulfuryldifluorid zu 1,2,2,2-Tetrafluor-1-(trifluormethoxy)-ethansulfonylfluorid weniger aufwendig und leichter handhabbar als eine aufwendige Umsetzung des schwer handhabbaren und schwer zugänglichen Trifluomethylhypofluorit, CF₃OF, mit Trifluorvinylsulfonylfluorid, CF₂=CFSO₂F. Darüber hinaus kann durch das erfindungsgemäße Verfahren eine größere Ausbeute erzielt werden.

Lithium-1-trifluormethoxy-1,2,2,2-tetrafluor-ethansulfonat ist als Leitsalz in einem Elektrolyten umfassend beispielsweise eine ionische Flüssigkeit in Lithium-basierten Energiespeichern verwendbar.

Ionische Flüssigkeiten sind als nicht-wässrige Elektrolyten insbesondere für elektrochemische Verwendungen beispielsweise in Batterien oder elektrochemischen Kondensatoren verwendbar, jedoch auch für die Elektroplattierung, Katalyse oder chemische Reaktionen. Ionische Flüssigkeiten mit vorzugsweise breitem elektrochemischem Fenster und geringer Hygroskopie sind entsprechend nicht nur für elektrochemische Anwendungen vorteilhaft verwendbar.

Ein weiterer Gegenstand der Erfindung betrifft eine ionische Flüssigkeit umfassend 1-Trifluormethoxy-1,2,2,2-tetrafluor-ethansulfonat und ein organisches Kation ausgewählt aus der Gruppe umfassend Alkylammonium-, Pyridinium-, Pyrazolium-, Pyrrolium-, Pyrrolinium-, Piperidinium-, Pyrrolidinium-, Imidazolium- und/oder Sulfoniumverbindungen. Vorzugsweise ist das organische Kation ausgewählt aus der Gruppe umfassend Alkylammonium-, Pyridinium-, Pyrazolium-, Pyrrolium-, Pyrrolinium-, Piperidinium-, Pyrrolidinium- und/oder Imidazoliumverbindungen, insbesondere Pyrrolidinium- und/oder Imidazoliumverbindungen. Vorzugsweise ist das Kation ausgewählt aus der Gruppe umfassend N-butyl-N-methylpyrrolidinium (PYR14), N-methyl-N-propylpyrrolidinium (PYR13), 1-Ethyl-3-methylimidazolium (EMIM), 1-Ethyl-2,3-dimethylimidazolium (EDiMIM) und/oder 1-Butyl-3-methylimidazolium (BMIM).

Ionische Flüssigkeiten umfassend 1-Trifluormethoxy-1,2,2,2-tetrafluor-ethansulfonat als Anion und ein organisches Kation können in vorteilhafter Weise für elektrochemische Anwendungen Verwendung finden, beispielsweise in Kombination mit einem Lithiumsalz in Lithium-basierten Energiespeichern. Weiterhin sind Verwendungen in Solarzellen oder Brennstoffzellen vorteilhaft. In vorteilhafter Weise sind ionische Flüssigkeiten umfassend fluorierte Anionen auch als Hydraulikflüssigkeit oder inerte flüssige Verdünnungsmittel für hochreaktive Chemikalien verwendbar.

Beispiele und Figuren, die der Veranschaulichung der vorliegenden Erfindung dienen, sind nachstehend angegeben.

Hierbei zeigen die Figuren:

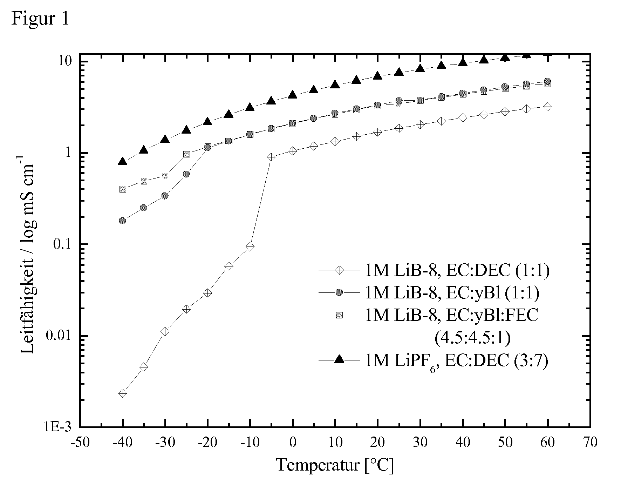

- Figur 1: zeigt die Leitfähigkeit einer 1 M Lösung von Lithium-1-trifluormethoxy-1,2,2,2-tetrafluor-ethansulfonat (LiB-8) in verschiedenen Lösungsmittelgemischen in Abhängigkeit von der Temperatur.

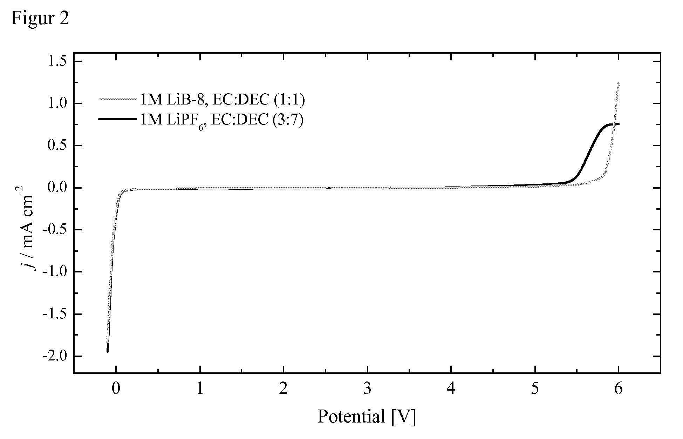

- Figur 2: zeigt das elektrochemische Stabilitätsfenster einer 1 M Lösung von Lithium-1-trifluormethoxy-1,2,2,2-tetrafluor-ethansulfonat (LiB-8) in einem Lösungsmittelgemisch aus Ethylencarbonat und Diethylcarbonat (EC:DEC) im Verhältnis 1:1 und von LiPF₆ in EC:DEC im Verhältnis 3:7. Aufgetragen ist die Stromdichte gegen das Potential.

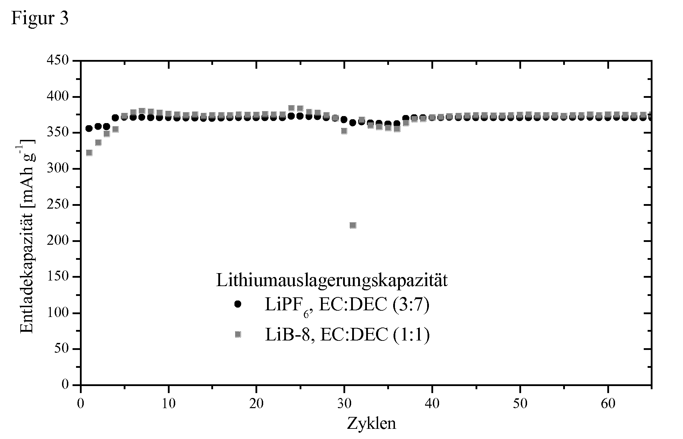

- Figur 3: zeigt die Entladekapazität einer Lithium-Ionen Halbzelle mit 1 M Lithium-1-trifluormethoxy-1,2,2,2-tetrafluor-ethansulfonat (LiB-8) in einem Lösungsmittelgemisch aus Ethylencarbonat und Diethylcarbonat (EC:DEC) im Verhältnis 1:1 und Graphit als Arbeitselektrode im Vergleich zu LiPF₆. Aufgetragen ist die Entladekapazität gegen die Zyklenzahl.

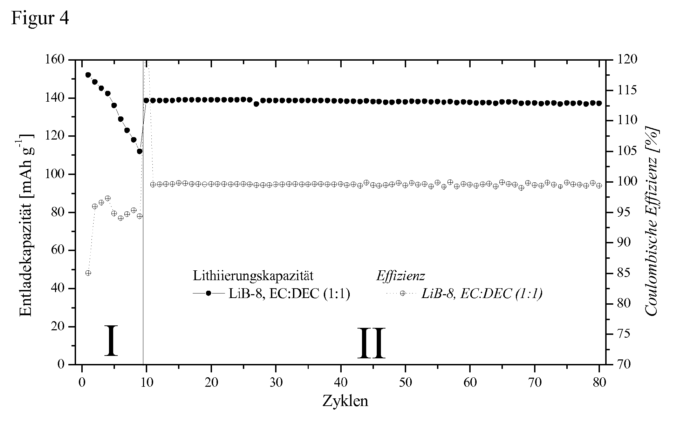

- Figur 4: zeigt die Entladekapazität und die Effizienz einer Lithium-Ionen Halbzelle mit 1 M Lithium-1-trifluormethoxy-1,2,2,2-tetrafluor-ethansulfonat (LiB-8) in einem Lösungsmittelgemisch aus Ethylencarbonat und Diethylcarbonat (EC:DEC) im Verhältnis 1:1. Als Arbeitselektrode diente das Kathodenmaterial Nickel-Mangan-Kobaltoxid. Aufgetragen ist die Entladekapazität gegen die Zyklenzahl.

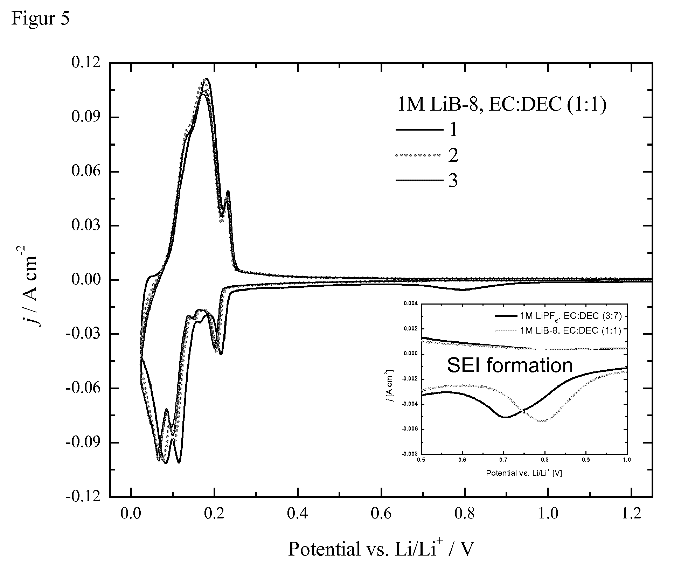

- Figur 5: zeigt die zyklische Voltammetrie einer 1 M Lösung von Lithium-1-trifluormethoxy-1,2,2,2-tetrafluor-ethansulfonat (LiB-8) in einem Lösungsmittelgemisch aus Ethylencarbonat und Diethylcarbonat (EC:DEC) im Verhältnis 1:1 auf einer Graphitanode für drei Zyklen (1), (2) und (3).

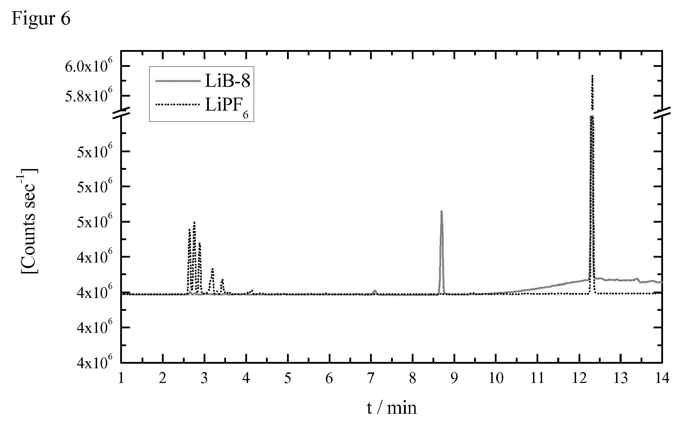

- Figur 6: zeigt die Zerfallsprodukte der thermischen Alterung bei 95°C einer 1 M Lösung von Lithium-1-trifluormethoxy-1,2,2,2-tetrafluor-ethansulfonat (LiB-8) in Ethylencarbonat und Diethylcarbonat (EC:DEC) im Verhältnis 1:1 im Vergleich zu LiPF₆ in EC:DEC im Verhältnis 3:7.

### Beispiel 1

Herstellung von Lithium-1-trifluormethoxy-1,2,2,2-tetrafluor-ethansulfonat
a) Herstellung von 1,2,2,2-Tetrafluor-1-(trifluormethoxy)-ethansulfonylfluorid 10 mg Tetramethylammoniumfluorid (ABCR) wurden in 10 ml trockenem Bis(2-methoxyethyl)ether (Diglyme, ABCR) suspendiert. Bei -197°C wurden 36 mmol Sulfuryldifluorid (ABCR) und 36 mmol 1,1,2-Trifluor-2-(trifluormethoxy)ethen (ABCR) aufkondensiert. Das Reaktionsgemisch wurde für 12 h auf 60°C erwärmt und anschließend das Produkt destilliert. 1,2,2,2-Tetrafluor-1-(trifluormethoxy)-ethansulfonylfluorid wurde als farblose Flüssigkeit in einer Ausbeute von 92 % erhalten.
b) Herstellung von Lithium-1,2,2,2-Tetrafluor-1-(trifluormethoxy)-ethansulfonat 20 mmol des in Schritt a) hergestellten 1,2,2,2-Tetrafluor-1-(trifluormethoxy)-ethansulfonylfluorids wurden in 10 ml Ethanol (ROTH) gelöst. Bei 0°C wurden 40 mmol Lithiumhydroxid (ROTH) zugegeben. Die Suspension wurde 2 h bei Raumtemperatur (20±3°C) gerührt, dann 15 min zentrifugiert und die flüssige Phase abdekantiert. Das Lösungsmittel wurde abgezogen und das Produkt im Vakuum (0,001mm) bei 60°C 6 h getrocknet. Die Ausbeute betrug 65 %.

Lithium-1-trifluormethoxy-1,2,2,2-tetrafluor-ethansulfonat wurde vor Gebrauch für 24 Stunden bei 60 °C getrocknet.

### Beispiel 2

### Bestimmung der Leitfähigkeit von Lithium-1-trifluormethoxy-1,2,2,2-tetrafluor-ethansulfonat

Die Leitfähigkeit von Lithium-1-trifluormethoxy-1,2,2,2-tetrafluor-ethansulfonat wurde in unterschiedlichen Lösungsmitteln in einem Temperaturbereich von - 40°C bis + 60°C bestimmt.

Mischungen von 50 Gew.-% Ethylencarbonat (EC) (Ferro Corporation, Batteriegrad) und 50 Gew.-% Diethylcarbonat (DEC) (Ferro Corporation, Batteriegrad) (EC:DEC, 1:1), von 50 Gew.-% Ethylencarbonat und 50 Gew.-% gamma-Butyrolacton (γBL) (Ferro Corporation, Batterygrade) (EC: γBL, 1:1), und von 45 Gew.-% Ethylencarbonat, 45 Gew.-% gamma-Butyrolacton und 10 Gew.-% Fluoroethylencarbonat (Solvay GmbH) (EC: γBL:FEC, 4,5:4,5:1) wurden vorgelegt. In diesen Lösungsmittelgemischen wurden pro Milliliter 217 mg des gemäß Beispiel 1 hergestellten Lithium-1-trifluormethoxy-1,2,2,2-tetrafluor-ethansulfonats gelöst, so dass sich eine Konzentration von 1 M ergab. Zum Vergleich wurde eine 1 M-Lösung von LiPF₆ (Sigma-Aldrich, Batteriegrad) in einem Gemisch von 30 Gew.-% Ethylencarbonat und 70 Gew.-% Diethylcarbonat (EC:DEC, 3:7) hergestellt.

Die Leitfähigkeit der Elektrolyten wurde unter Verwendung von Platin-Leitfähigkeitsmesszellen (Amel Glassware, Zellkonstante 1 cm⁻¹) mit einem Potentiostaten (Solartron 1287A) in Verbindung mit einer Impedanzmesseinheit (Solartron 1260) in einem Temperaturbereich von - 40°C bis + 60°C (Klimaschrank, Binder MK53) untersucht. Hierzu wurden die Leitfähigkeitsmesszellen zunächst auf 60°C erwärmt und dann in Temperaturintervallen von 5°C auf -40°C gekühlt.

Die Figur 1 zeigt den Verlauf der Leitfähigkeit der 1 M Lösungen von Lithium-1-trifluormethoxy-1,2,2,2-tetrafluor-ethansulfonat (LiB-8) in den verschiedenen Lösungsmittelgemischen im Temperaturbereich von -40°C bis +60°C sowie die von LiPF₆. Wie man der Figur 1 entnehmen kann, war die Leitfähigkeit in den Lösungsmittelgemischen Ethylencarbonat und gamma-Butyrolacton (EC: γBL, 1:1) und Ethylencarbonat, gamma-Butyrolacton und Fluoroethylencarbonat (EC:γBL:FEC) im Temperaturbereich von - 40°C bis + 0°C deutlich höher gegenüber der Leitfähigkeit in Ethylencarbonat und Diethylcarbonat (EC:DEC, 1:1) und erreichte über den gesamten Temperaturbereich nahezu die Leitfähigkeit von LiPF₆ in einem Ethylencarbonat und Diethylcarbonat (EC:DEC, 3:7).

Die Leitfähigkeit von 1 M Lithium-1-trifluormethoxy-1,2,2,2-tetrafluor-ethansulfonat bei 25°C betrug in den verschiedenen Lösungsmittelgemischen für ein 1:1-Gemisch Ethylencarbonat und Diethylcarbonat (EC:DEC 1:1) 1,8 mS cm⁻¹, für ein 1:1-Gemisch Ethylencarbonat und gamma-Butyrolacton (EC: γBL, 1:1) 3,7 mS cm⁻¹ und für ein Gemisch Ethylencarbonat, gamma-Butyrolacton und Fluoroethylencarbonat (EC:γBL:FEC) im Verhältnis 4,5:4,5:1 3,5 mS cm⁻¹.

Dies zeigt, dass Lithium-1-trifluormethoxy-1,2,2,2-tetrafluor-ethansulfonat in den üblichen Carbonat-Lösungsmitteln eine ausreichende Leitfähigkeit bei 25°C aufweist.

### Beispiel 3

### Bestimmung der elektrochemischen Stabilität von Lithium-1-trifluormethoxy-1,2,2,2-tetrafluor-ethansulfonat

Die elektrochemische Stabilität einer 1 M-Lösung von Lithium-1-trifluormethoxy-1,2,2,2-tetrafluor-ethansulfonat hergestellt gemäß Beispiel 1 in einem Lösungsmittelgemisch aus 50 Gew.-% Ethylencarbonat und 50 Gew.-% Diethylcarbonat (EC:DEC, 1:1) gegenüber der Stabilität einer 1 M-Lösung von LiPF₆ (Sigma-Aldrich, Batteriegrad) in einem Gemisch von 30 Gew.-% Ethylencarbonat und 70 Gew.-% Diethylcarbonat (EC:DEC, 3:7) wurde mittels der sogenannten Linear Sweep-Voltammetrie (LSV) bestimmt. Bei diesem Verfahren erfolgt eine kontinuierliche Änderung der Elektrodenspannung (linear sweep).

Als kathodisches Stabilitätslimit, das Potential, bei welchem eine Reduktion einsetzt, wurde jenes Potential definiert, bei welchem die Stromdichte - 0,1 mA cm⁻² unterschritt. Als anodisches Stabilitätslimit, das Potential, bei welchem eine Oxidation einsetzt, wurde jenes Potential definiert, bei welchem die Stromdichte + 0,1 mA cm⁻² überschritt. Insbesondere die anodische Stabilität hängt entscheidend von der Stabilität des verwendeten Elektrolyten ab.

Die Versuche wurden in einer 3-Elektrodenanordnung in modifizierte Swagelok®-T-Stücke (Rohrverbinder, Edelstahlkörper) mit einer Platinelektrode (eDAQ, Modell ET075, 1 mm Durchmesser) als Arbeitselektrode und Lithiumfolie (12 mm bzw. 7 mm Durchmesser, Chemetall) als Gegen- und Referenzelektrode durchgeführt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war. Die Vorschubgeschwindigkeit betrug 1 mV s⁻¹.

Wie in Figur 2 gezeigt ist, wurde bei dem Elektrolyten 1 M Lithium-1-trifluormethoxy-1,2,2,2-tetrafluor-ethansulfonat (LiB-8) in einer 1:1 Mischung von Ethylencarbonat und Diethylcarbonat das kathodische Stabilitätslimit bei 0,03 V erreicht. Die anodische Stabilität von Lithium-1-trifluormethoxy-1,2,2,2-tetrafluor-ethansulfonat betrug 5,7 V und ist völlig ausreichend für eine Verwendung des Elektrolyten in Kombination mit Hochvoltkathodenmaterialien.

Dieses Ergebnis zeigt, dass Lithium-1-trifluormethoxy-1,2,2,2-tetrafluor-ethansulfonat in den üblichen Carbonat-Lösungsmitteln eine für sämtliche elektrochemischen Anwendungen ausreichend gute elektrochemischen Stabilität aufweist.

### Beispiel 4

### Bestimmung des Zyklisierverhaltens von Lithium-1-trifluormethoxy-1,2,2,2-tetrafluor-ethansulfonat mit Graphitelektrode

Das Zyklisierverhalten einer 1 M-Lösung von Lithium-1-trifluormethoxy-1,2,2,2-tetrafluor-ethansulfonat hergestellt gemäß Beispiel 1 wurde in einem Gemisch von 50 Gew.-% Ethylencarbonat und 50 Gew.-% Diethylcarbonat (EC:DEC, 1:1) gegenüber der Zyklisierung einer 1 M-Lösung von LiPF₆ in einem Gemisch von 30 Gew.-% Ethylencarbonat und 70 Gew.-% Diethylcarbonat (EC:DEC, 3:7) bestimmt.

Die Versuche wurden in einer 3-Elektrodenanordnung in modifizierte Swagelok®-T-Stücke (Rohrverbinder, Edelstahlkörper) mit einer Graphitelektrode (Timcal T44 Graphitmaterial) als Arbeitselektrode und Lithiumfolie (12 mm bzw. 5 mm Durchmesser, Chemetall) als Gegen- und Referenzelektrode durchgeführt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war.

Der Test zum Zyklisierverhalten umfasste mehrere Phasen. In der ersten Phase wurde die Formierung des Graphits (SEI Formation) durch drei Zyklen mit einer Konstantstrom C-Rate von C/5 sichergestellt. Im Anschluss wurde in der zweiten Phase über 20 Zyklen das Zyklisierverhalten bei einer Lade- und Entladerate von 1C überprüft. Dabei wurde das Zellsystem nach dem Laden für eine Stunde bei einer Spannung von 0,025 V gehalten. In der dritten Phase wurde der Graphit immer mit C/2 geladen und im Anschluss für eine Stunde bei 0,025 V gehalten bevor der Graphit mit unterschiedlichen Geschwindigkeiten entladen wurde. Die angewendeten D-Raten (Entladeraten) waren dabei D/5, D/3; D/2, 1D, 2D, 3D, 5D und 10D. Dem D-Ratentest folgten fünf Zyklen mit Lade- und Entladeraten von C/5, um zu überprüfen ob der Graphit durch den Belastungstest Schaden genommen hatte. Die letzte Phase beinhaltete die gleichen Zyklisierparameter wie Phase 2 und wurde für 30 Zyklen durchgeführt.

In der Figur 3 sind die Entladekapazität bzw. Lithiumauslagerungskapazität der 1 M-Lösungen von Lithium-1-trifluormethoxy-1,2,2,2-tetrafluor-ethansulfonat und LiPF₆ gegen die Zyklenzahl der mit einer C-Rate von 1C geladenen Lithiumionen-Batteriehalbzelle dargestellt. Wie die Figur 3 zeigt, wies die Halbzelle mit 1 M Lithium-1-trifluormethoxy-1,2,2,2-tetrafluor-ethansulfonat nach dem Formieren der Zelle eine Anfangskapazität von ca. 373 mAh g⁻¹ auf, die mit der Anzahl der Zyklen auf ca. 374 mAh g⁻¹ im 15. Zyklus stieg. Dies zeigt die hervorragende Zyklenstabilität der Zelle, die der von LiPF₆ entspricht.

### Beispiel 5

### Bestimmung des Zyklisierverhaltens von Lithium-1-trifluormethoxy-1,2,2,2-tetrafluor-ethansulfonat mit NCM-Kathode

Das Zyklisierverhalten auf NCM-Kathoden in einer Halbzelle wurde wie in Beispiel 4 dargestellt in einer 3-Elektrodenanordnung durchgeführt, wobei eine Nickel-Cobalt-Manganoxid-Elektrode (NCM-Elektrode, Toda Kogyo Europe GmbH) als Arbeitselektrode und Lithiumfolie (12 mm bzw. 5 mm Durchmesser, Chemetall) als Gegen- und Referenzelektrode verwendet wurde. Hierbei wurde eine 1 M-Lösung von Lithium-1-trifluormethoxy-1,2,2,2-tetrafluor-ethansulfonat hergestellt gemäß Beispiel 1 in einem Gemisch von 50 Gew.-% Ethylencarbonat und 50 Gew.-% Diethylcarbonat (EC:DEC, 1:1) verwendet.

Die Figur 4 zeigt die Entladekapazität bzw. Lithiumeinlagerungskapazität und die Effizienz der Lithium-Ionen Halbzelle. Aufgetragen ist die Ladekapazität und die Effizienz gegen die Zyklenzahl. Wie die Figur 4 zeigt, wies die Halbzelle mit 1 M Lithium-1-trifluormethoxy-1,2,2,2-tetrafluor-ethansulfonat eine Anfangskapazität von ca. 138 mAh g⁻¹ nach dem erfolgreich bestandenen Stromraten-Belastungstest (C-Ratentest) der Zelle (I) auf. Nach der nachfolgenden Zyklisierung (II) lag die Kapazität im 80. Zyklus noch bei ca. 137 mAh g⁻¹. Weiterhin stieg die Effizienz der Zelle nach 2 Standardzyklen auf über 99,6 % einer maximalen Effizienz von 100 %. Somit konnte festgestellt werden, dass die Zelle auch mit NCM-Kathode eine hervorragende Zyklenstabilität zeigte.

### Beispiel 6

### Zyklische Voltammetrie

Die zyklische Voltammetrie einer 1 M-Lösung von Lithium-1-trifluormethoxy-1,2,2,2-tetrafluor-ethansulfonat hergestellt gemäß Beispiel 1 wurde in einem Gemisch von 50 Gew.-% Ethylencarbonat und 50 Gew.-% Diethylcarbonat (EC:DEC, 1:1) durchgeführt.

Die Versuche wurden in einer 3-Elektrodenanordnung in modifizierte Swagelok®-T-Stücke (Rohrverbinder, Edelstahlkörper) mit einer Graphitelektrode (Timcal T44 Graphitmaterial) als Arbeitselektrode und Lithiumfolie (12 mm bzw. 4 mm Durchmesser, Chemetall) als Gegen- und Referenzelektrode durchgeführt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war.

Die Figur 5 zeigt die Ergebnisse der zyklischen Voltammetrie für drei Zyklen (1), (2) und (3). In der Figur 5 sind die durch die erhöhte Stromdichte identifizierbaren Interkalations- und Deinterkalationsphasen in den jeweiligen Zyklen im Bereich zwischen 0 und 0,3 V erkennbar. Dies belegt die Reversibilität des Systems.

Der vergrößerte Ausschnitt des Bereichs von 0,5 V bis 1 V zeigt die Bildung der Solid Electrolyte Interphase (SEI) des Lithium-1-trifluormethoxy-1,2,2,2-tetrafluor-ethansulfonat-Carbonat Elektrolyten. Im Vergleich dazu ist das Entstehungspotential der SEI für eine 1 M-Lösung von LiPF₆ in einem Gemisch von 30 Gew.-% Ethylencarbonat und 70 Gew.-% Diethylcarbonat (EC:DEC, 3:7) gezeigt.

### Beispiel 7

### Thermische Stabilität

Die Zerfallsprodukte einer 1 M-Lösung von Lithium-1-trifluormethoxy-1,2,2,2-tetrafluor-ethansulfonat hergestellt gemäß Beispiel 1 in einem Gemisch von 50 Gew.-% Ethylencarbonat und 50 Gew.-% Diethylcarbonat (EC:DEC, 1:1) wurden gegenüber den Zerfallsprodukten einer 1 M-Lösung von LiPF₆ (Sigma-Aldrich, Batteriegrad) in einem Gemisch von 30 Gew.-% Ethylencarbonat und 70 Gew.-% Diethylcarbonat (EC:DEC, 3:7) bestimmt.

Die Elektrolyte wurden in einer Klimakammer für zwei Wochen bei 95 °C gelagert und im Anschluss mittels einer Gaschromatographie-Massenspektrometrie (Clarus 600 GC von Perkin Elmer) untersucht.

Figur 6 zeigt die Zerfallsprodukte der thermischen Alterung bei 95°C einer 1 M Lösung von Lithium-1-trifluormethoxy-1,2,2,2-tetrafluor-ethansulfonat (LiB-8) in Ethylencarbonat und Diethylcarbonat (EC:DEC) im Verhältnis 1:1 im Vergleich zu 1 M LiPF₆ in EC:DEC im Verhältnis 3:7. Wie die Figur 6 zeigt, wurden für den Elektrolyten 1 M Lithium-1-trifluormethoxy-1,2,2,2-tetrafluor-ethansulfonat in EC:DEC mittels der GC-MS keine Carbonatzersetzungsprodukte in dem thermisch gealtertem Elektrolyten gefunden. Demgegenüber wurden für den Elektrolyten 1 M LiPF₆ in EC:DEC anhand der Signale zwischen 2,5 min und 3,5 min Carbonat-Zersetzungsprodukte detektiert. Das Signal nach 12 min ist Diethylcarbonat zuzuordnen. Dies zeigt, dass die thermische Stabilität von 1 M Lithium-1-trifluormethoxy-1,2,2,2-tetrafluor-ethansulfonat die Stabilität des LiPF₆ Elektrolyten überstieg.

Diese Ergebnisse zeigen insgesamt, dass Lithium-1-trifluormethoxy-1,2,2,2-tetrafluor-ethansulfonat eine Möglichkeit bietet, LiPF₆ als Leitsalz in Lithium-Ionen Batterien zu ersetzen.

## Patentansprüche

1. Lithium-1-trifluormethoxy-1,2,2,2-tetrafluor-ethansulfonat.

2. Verwendung von Lithium-1-trifluormethoxy-1,2,2,2-tetrafluor-ethansulfonat als Leitsalz in Lithium-basierten Energiespeichern.

3. Elektrolyt für einen Lithium-basierten Energiespeicher umfassend Lithium-1-trifluormethoxy-1,2,2,2-tetrafluor-ethansulfonat.

4. Elektrolyt nach Anspruch 3, **dadurch gekennzeichnet, dass** der Elektrolyt ein organisches Lösungsmittel, eine ionische Flüssigkeit und/oder eine Polymermatrix umfasst.

5. Elektrolyt nach Anspruch 3 oder 4, **dadurch gekennzeichnet, dass** das organische Lösungsmittel ausgewählt ist aus der Gruppe umfassend Ethylencarbonat, Propylencarbonat, Diethylcarbonat, Dimethylcarbonat, Ethylmethylcarbonat, Acetonitril, Glutaronitril, Adiponitril, Pimelonitril, gamma-Butyrolacton, gamma-Valerolacton, Dimethoxyethan, 1,3-Dioxolan, Methylacetat und/oder Mischung davon, vorzugsweise aus der Gruppe umfassend Ethylencarbonat, Diethylcarbonat, Dimethylcarbonat und/oder deren Mischungen.

6. Elektrolyt nach einem der Ansprüche 3 bis 5, **dadurch gekennzeichnet, dass** das organische Lösungsmittel eine Mischung aus Ethylencarbonat und wenigstens einem weiteren organischen Lösungsmittel vorzugsweise ausgewählt aus der Gruppe umfassend gamma-Butyrolacton und/oder Diethylcarbonat umfasst, bevorzugt im Verhältnis im Bereich von ≥ 1:99 bis ≤ 99:1, bevorzugt im Bereich von ≥ 1:9 bis ≤ 9:1, vorzugsweise im Bereich von ≥ 3:7 bis ≤ 1:1.

7. Elektrolyt nach einem der Ansprüche 3 bis 6 , **dadurch gekennzeichnet, dass** die Konzentration von Lithium-1-trifluormethoxy-1,2,2,2-tetrafluor-ethansulfonat im Elektrolyten im Bereich von ≥ 0,1 M bis ≤ 2 M, vorzugsweise im Bereich von ≥ 0,5 M bis ≤ 1,5 M, besonders bevorzugt im Bereich von ≥ 0,7 M bis ≤ 1,2 M, liegt.

8. Lithium-basierter Energiespeicher, vorzugsweise Lithium-Batterie, Lithium-Ionen-Batterie, Lithium-Ionen-Akkumulator, Lithium-Polymer-Batterie oder Lithium-Ionen-Kondensator, umfassend Lithium-1-trifluormethoxy-1,2,2,2-tetrafluor-ethansulfonat.

9. Verfahren zur Herstellung von Lithium-1-trifluormethoxy-1,2,2,2-tetrafluor-ethansulfonat, umfassend die folgenden Schritte:
a) Umsetzung von 1,1,2-Trifluor-2-(trifluormethoxy)ethen mit Sulfuryldifluorid zu 1,2,2,2-Tetrafluor-1-(trifluormethoxy)-ethansulfonylfluorid, und
b) Umsetzung von 1,2,2,2-Tetrafluor-1-(trifluormethoxy)-ethansulfonylfluorid mit Lithiumhydroxid zu Lithium-1-trifluormethoxy-1,2,2,2-tetrafluor-ethansulfonat.

10. Ionische Flüssigkeit umfassend 1-Trifluormethoxy-1,2,2,2-tetrafluor-ethansulfonat und ein organisches Kation ausgewählt aus der Gruppe umfassend Alkylammonium-, Pyridinium-, Pyrazolium-, Pyrrolium-, Pyrrolinium-, Piperidinium-, Pyrrolidinium-, Imidazolium- und/oder Sulfoniumverbindungen, wobei das Kation vorzugsweise ausgewählt ist aus der Gruppe umfassend N-butyl-N-methylpyrrolidinium, N-methyl-N-propylpyrrolidinium, 1-Ethyl-3-methylimidazolium, 1-Ethyl-2,3-dimethylimidazolium und/oder 1-Butyl-3-methylimidazolium.

## Claims

1. Lithium 1-trifluoromethoxy-1,2,2,2-tetrafluoro-ethanesulfonate.

2. Use of lithium 1-trifluoromethoxy-1,2,2,2-tetrafluoroethanesulfonate as conductive salt in lithium-based energy storage means.

3. Electrolyte for a lithium-based energy storage means comprising lithium 1-trifluoromethoxy-1,2,2,2-tetrafluoroethanesulfonate.

4. Electrolyte according to Claim 3, **characterized in that** the electrolyte comprises an organic solvent, an ionic liquid and/or a polymer matrix.

5. Electrolyte according to Claim 3 or 4, **characterized in that** the organic solvent is selected from the group comprising ethylene carbonate, propylene carbonate, diethyl carbonate, dimethyl carbonate, ethyl methyl carbonate, acetonitrile, glutaronitrile, adiponitrile, pimelonitrile, gamma-butyrolactone, gamma-valerolactone, dimethoxyethane, 1,3-dioxolane, methyl acetate and/or a mixture thereof, preferably from the group comprising ethylene carbonate, diethyl carbonate, dimethyl carbonate and/or mixtures thereof.

6. Electrolyte according to any of Claims 3 to 5, **characterized in that** the organic solvent comprises a mixture of ethylene carbonate and at least one further organic solvent preferably selected from the group comprising gamma-butyrolactone and/or diethyl carbonate, preferably in a ratio in the range from ≥ 1:99 to ≤ 99:1, preferably in the range from ≥ 1:9 to ≤ 9:1, preferably in the range from ≥ 3:7 to ≤ 1:1.

7. Electrolyte according to any of Claims 3 to 6, **characterized in that** the concentration of lithium 1-trifluoromethoxy-1,2,2,2-tetrafluoroethane-sulfonate in the electrolyte is in the range from ≥ 0.1 M to ≤ 2 M, preferably in the range from ≥ 0.5 M to ≤ 1.5 M, more preferably in the range from ≥ 0.7 M to ≤ 1.2 M.

8. Lithium-based energy storage means, preferably lithium battery, lithium ion battery, lithium ion accumulator, lithium polymer battery or lithium ion capacitor, comprising lithium 1-trifluoromethoxy-1,2,2,2-tetrafluoroethanesulfonate.

9. Process for preparing lithium 1-trifluoro-methoxy-1,2,2,2-tetrafluoroethanesulfonate, comprising the following steps:
a) reacting 1,1,2-trifluoro-2-(trifluoromethoxy)ethene with sulfuryl difluoride to give 1,2,2,2-tetra-fluoro-1-(trifluoromethoxy)ethanesulfonyl fluoride, and
b) reacting 1,2,2,2-tetrafluoro-1-(trifluoromethoxy)-ethanesulfonyl fluoride with lithium hydroxide to give lithium 1-trifluoromethoxy-1,2,2,2-tetra-fluoroethanesulfonate.

10. Ionic liquid comprising 1-trifluoro-methoxy-1,2,2,2-tetrafluoroethanesulfonate and an organic cation selected from the group comprising alkylammonium, pyridinium, pyrazolium, pyrrolium, pyrrolinium, piperidinium, pyrrolidinium, imidazolium and/or sulfonium compounds, where the cation is preferably selected from the group comprising N-butyl-N-methylpyrrolidinium, N-methyl-N-propyl-pyrrolidinium, 1-ethyl-3-methylimidazolium, 1-ethyl-2,3-dimethylimidazolium and/or 1-butyl-3-methylimidazolium.

## Revendications

1. 1-Trifluorométhoxy-1,2,2,2-tétrafluoro-éthane-sulfonate de lithium.

2. Utilisation du 1-trifluorométhoxy-1,2,2,2-tétrafluoro-éthanesulfonate de lithium comme sel conducteur dans des accumulateurs d'énergie à base de lithium.

3. Électrolyte pour un accumulateur d'énergie à bas de lithium, comprenant du 1-trifluorométhoxy-1,2,2,2-tétrafluoro-éthanesulfonate de lithium.

4. Électrolyte selon la revendication 3, **caractérisé en ce que** l'électrolyte comprend un solvant organique, un liquide ionique et/ou une matrice polymère.

5. Électrolyte selon la revendication 3 ou 4, **caractérisé en ce que** le solvant organique est choisi dans le groupe comprenant le carbonate d'éthylène, le carbonate de propylène, le carbonate de diéthyle, le carbonate de diméthyle, le carbonate d'éthyle et de méthyle, l'acétonitrile, le glutaronitrile, l'adiponitrile, le pimélonitrile, la gamma-butyrolactone, la gamma-valérolactone, le diméthoxyéthane, le 1,3-dioxolane, l'acétate de méthyle et/ou un mélange de ceux-ci, de préférence dans le groupe comprenant le carbonate d'éthylène, le carbonate de diéthyle, le carbonate de diméthyle et/ou des mélanges de ceux-ci.

6. Électrolyte selon l'une quelconque des revendications 3 à 5, **caractérisé en ce que** le solvant organique comprend un mélange de carbonate d'éthylène et d'au moins un autre solvant organique de préférence choisi dans le groupe comprenant la gamma-butyrolactone et/ou le carbonate de diéthyle, de préférence en un rapport dans la plage de ≥ 1:99 à ≤ 99:1, de préférence dans la plage de ≥ 1:9 à ≤ 9:1, encore mieux dans la plage de ≥ 3:7 à ≤ 1:1.

7. Électrolyte selon l'une quelconque des revendications 3 à 6, **caractérisé en ce que** la concentration de 1-trifluorométhoxy-1,2,2,2-tétrafluoro-éthanesulfonate de lithium dans l'électrolyte se situe dans la plage de ≥ 0,1 M à ≤ 2 M, de préférence dans la plage de ≥ 0,5 M à ≤ 1,5 M, de façon particulièrement préférée dans la plage de ≥ 0,7 M à ≤ 1,2 M.

8. Accumulateur d'énergie à base de lithium, de préférence batterie lithium, batterie lithium-ion, accumulateur lithium-ion, batterie lithium-polymère ou condensateur lithium-ion, comprenant du 1-trifluoro-méthoxy-1,2,2,2-tétrafluoro-éthanesulfonate de lithium.

9. Procédé pour la préparation de 1-trifluoro-méthoxy-1,2,2,2-tétrafluoro-éthanesulfonate de lithium, comprenant les étapes suivantes :
a) mise en réaction de 1,1,2-trifluoro-2-(trifluorométhoxy)éthene avec du fluorure de sulfuryle pour l'obtention de fluorure de 1,2,2,2-tétrafluoro-1-(trifluorométhoxy)-éthanesulfonyle, et
b) mise en réaction de fluorure de 1,2,2,2-tétrafluoro-1-(trifluorométhoxy)-éthanesulfonyle avec de l'hydroxyde de lithium pour l'obtention de 1-trifluorométhoxy-1,2,2,2-tétrafluoroéthane-sulfonate de lithium.

10. Liquide ionique comprenant du 1-trifluoro-méthoxy-1,2,2,2-tétrafluoro-éthanesulfonate et un cation organique choisi dans le groupe comprenant des composés alkylammonium, pyridinium, pyrazolium, pyrrolium, pyrrolinium, pipéridinium, pyrrolidinium, imidazolium et/ou sulfonium, le cation étant de préférence choisi dans le groupe comprenant le N-butyl-N-méthylpyrrolidinium, le N-méthyl-N-propyl-pyrrolidinium, le 1-éthyl-3-méthylimidazolium, le 1-éthyl-2,3-diméthylimidazolium et/ou le 1-butyl-3-méthylimidazolium.
